# EUROPEAN PATENT APPLICATION

(11) **EP 4 338 771 A1**
(43) Date of publication of application: **20.03.2024**
(21) Application number: 22382853.4
(22) Date of filing: 16.09.2022
(51) Int. Cl.: A61M 5/168, A61M 39/28

(54) **DEVICE FOR REGULATING THE FLUID FLOW RATE OF A DRIP**

(71) Applicant: Cabello Rey, Andrés, 35002 Las Palmas de Gran Canarias Las Palmas (ES); ID Mirai, S.L., 35002 Las Palmas de Gran Canaria (ES)
(72) Inventor: CABELLO REY, Andrés, 35002 Las Palmas de Gran Canarias Las Palmas (ES); GRASES MENDOZA, José Manuel, 28660 Boadilla del monte, Madrid (ES)
(74) Representative: ABG Intellectual Property Law, S.L.

(57) **Abstract**

The present invention relates to a device for regulating the fluid flow rate of a drip used in the medical field. The drip for which the regulator is intended comprises an elastically deformable duct, made typically of silicone, such that the drip is interposed at a point of the duct so as to allow visualizing the flow rate depending on the rate of fall of a droplet. The object of the invention relates to a regulator which receives a segment of the duct to establish a preestablished flow rate without this flow rate varying over time as a result of changes in the conditions of interaction between the duct and the regulator.

## Description

### OBJECT OF THE INVENTION

The present invention relates to a device for regulating the fluid flow rate of a drip used in the medical field. The drip for which the regulator is intended comprises an elastically deformable duct, made typically of silicone, such that the drip is interposed at a point of the duct so as to allow visualizing the flow rate depending on the rate of fall of a droplet.

The object of the invention relates to a regulator which receives a segment of the duct in order to establish a preestablished flow rate without this flow rate varying over time as a result of changes in the conditions of interaction between the duct and the regulator.

### BACKGROUND OF THE INVENTION

One of the fields of the art undergoing the most extensive development is the medical field. However, there are tools and devices commonly used in a hospital setting which have not seen improvements for a quite some time despite having certain drawbacks. One of these elements are drip flow regulators for the delivery of a liquid, such as serum or a medicinal product, intravenously.

The form of delivery of a liquid, such as serum or a drug stored in a reservoir, which can be a bottle or a bag, usually consists of positioning the liquid reservoir in a raised position and communicating the reservoir with the delivery line inserted into the vein by means of a silicone duct. The height difference between the reservoir and the point of insertion of the line generates pressure in the liquid which forces entry into the vein. The flow rate delivered to the patient will therefore depend on the resistance the liquid encounters in the passage thereof between the reservoir and the line.

Today, a common way of regulating flow rate consists of coupling to the silicone duct a small flow rate regulator which incorporates a flat seat or support for a segment of the duct and an essentially cylindrical-shaped body which can slide along a guide extending according to an oblique direction with respect to the support seat.

This cylindrical body can move between two end positions, a first end position which is not in contact with the duct and allows insertion or assembly thereof, and another end position in which the cylindrical body presses on the duct, such that it is not possible for the liquid to pass through the duct as a result of the deformation generated which constricts the inner passage. Any intermediate position of the cylindrical body establishes a degree of constriction of the elastically deformable duct which determines an also intermediate flow rate between the maximum flow rate and zero flow rate.

Once the desired flow rate has been established by positioning the cylindrical body in an intermediate position, said position is maintained by the elastic recovery force of the duct against the guide in combination with the friction forces between each of the elements: the guide, the cylindrical body, and the surface of the elastically deformable duct.

However, with one of the elements being elastically deformable and with the configuration that is described, what happens in practice is that the cylindrical body, in its wedging condition, gradually moves against the elastically deformable duct given that the shape thereof continues to change slowly and to give. As it gives, the regulator allows the passage of a greater flow rate which translates to a liquid delivery greater than the preestablished delivery.

There are drugs which require very precise dosing, and it is not acceptable for a regulator to have a previously adjusted flow that varies over time.

Such a regulator is still widely used due to its low manufacturing cost given its simplicity, and due to that fact the problem of the delivered flow rate lacking in stability over time with respect to the preestablished flow rate is compensated for with periodic inspections by the personnel in charge of the patient who readjust the regulator.

There are alternatives based on motor-driven delivery pumps for cases in which a very precise flow rate is needed and in which regulation is carried out on the motor operation. These devices are extremely expensive compared to the regulators described above and it is not possible to establish such a widespread use unless a very high cost is incurred.

With respect to flow rate measurement, the drip allows estimating the flow rate. All the liquid delivered is transferred through the drip such that the passage from the inlet to the outlet is always through a point where the liquid forms droplets that fall sequentially one by one. The size of the droplet depends on the liquid given that it depends mainly on the surface tension and the density of the liquid. Therefore, once the liquid is selected, it is possible to establish a ratio between the rate of fall of droplets in the drip and the flow rate which is being delivered.

There are devices for measuring flow rate, for example optical devices, which count the droplets of the drip such that they visually provide, for example through a screen, continuous information about the evolution of the flow rate.

By positioning such a device in the drip and using a reliable regulator such as the regulator of the invention, a very precise flow rate can be established for the patient using a regulator having a very low cost, which overcomes the drawbacks that have been described.

### DESCRIPTION OF THE INVENTION

A first aspect of the invention relates to a device for regulating the fluid flow rate of a drip, wherein the drip comprises an elastically deformable duct through which the fluid flow rate flows.

*The device comprises:*
- *a casing, comprising a channel extending along a first direction, configured for housing the duct therein in the operating mode,*
- *a press element, movable in a second direction, preferably transverse to the first direction, wherein the press element is configured for pressing on at least one part of the duct when the duct is arranged in the operating mode inside the channel, and*
- *a regulator element, connected to the press element, wherein the regulator element is configured for regulating the position of the press element in the second direction.*

The drip comprises an elastically deformable duct and the device acts on this elastically deformable duct, regulating flow by constricting the duct to a greater or lesser extent by means of a combination of elements which allow maintaining the regulation condition in a stable manner regardless of the recovery tendency of the elastically deformable duct.

The device comprises a casing which, in the embodiments, stores the set of components which allow regulation. This casing has a channel for the passage of the elastically deformable duct. Assembling the regulator on the elastically deformable duct consists of inserting the duct into the channel. The direction of the channel dictates the direction of the elastically deformable duct. This direction will be referred to as the first direction.

In the preferred embodiment, the channel is straight, and therefore the segment of the inserted duct also adopts a straight position, with the exception of small deformations that it may sustain due to the presence retaining protrusions or other elements characteristic of specific embodiments. However, according to other embodiments, it is possible for the channel to include curved inlets or outlets, in which case the direction defined by the direction of the channel where the elastically deformable duct is constricted shall be interpreted as the first direction.

The device further comprises a press element, which is responsible for providing a certain degree of constriction on the elastically deformable duct. The press element is movable in a second direction, different from the first direction. The preferred second direction is transverse to the first direction.

The elastically deformable duct is constricted by means of a press element resting on at least one part of the elastically deformable duct. The press element is movable according to the second direction and its position is determined by a regulator element connected to the press element which is configured for regulating its position. The degree of constriction is greater or lesser depending on the position adopted by the press element according to the second direction.

The functions of the press element and the regulator element being independent allows the position to be fixed once the regulator element establishes this position, and therefore the deformation of the elastically deformable duct does not vary over time, resulting in a reliable device.

According to one embodiment, *the device is characterized in that the press element is adapted, in the operating mode, to press on at least one part of the duct and wherein the channel comprises a support surface for the part located opposite the pressed part of the duct, when the duct is arranged in the operating mode in the channel, to cause a reduction in the section of the duct depending on the degree by which the press element and the support surface approach one another.*

According to this embodiment applicable to the examples describe above, the duct extends along the channel, being arranged in one of the segments thereof interposed between the press element and a support surface corresponding to a region of the surface of the channel. When the press element moves along the second direction approaching the support surface, the interposed duct is pressed on and compressed. Given that the duct is elastically deformable, constriction occurs, reducing the section area for the passage of fluid, and therefore reducing the flow rate. The flow rate that can go through the duct is established by determining the degree of constriction of the duct.

According to another embodiment applicable to the examples describe above, *the device is characterized in that the press element is arranged in an intermediate position of the casing with respect to the first direction in order to press, in the operating mode, on an intermediate part of the duct which is arranged in the channel.*

According to this example, the press element is in an intermediate position according to the first direction. That is, the channel in the casing houses a portion of the elastically deformable duct. The intermediate position of the press element establishes a first sub-segment on one side of the press element and a second sub-segment on the other side of the press element, such that there is a sub-segment housed in the channel on either side of the press element. This configuration allows a stable extended position of the elastically deformable duct although the press element causes deformation in one of its parts, causing a tendency to bend.

According to another embodiment applicable to the examples describe above, *the device is characterized in that the channel comprises an inner area for the seating of the duct when the duct is housed in the operating mode in the channel and an open outer area forming an opening adapted to receive the duct by insertion.*

According to this embodiment, the channel is an open channel which allows insertion through the open outer area. Another example would a channel which is closed at least in one segment but requires the passage of the conduit by introducing it longitudinally, which is not possible when it is already connected to the drip and to the line.

According to this example, the regulator device is coupled to the duct by insertion of a segment of the duct into the channel through the open outer area, resulting in a quick installation.

According to another embodiment applicable to the examples describe above, *the device is characterized in that the channel comprises in the open outer area two facing surfaces parallel to one another, arranged at a distance, and wherein the facing surfaces are provided with a plurality of protrusions distributed according to the first direction for the correct positioning and retention of the duct.*

According to this embodiment, the channel has an open area which allows the insertion and exit of the duct inside the channel so that the regulator can act on the duct. According to a preferred example, the casing has a prismatic body with two main faces, with the channel being open towards one of said main faces. In this embodiment, the outer area is interpreted as the area corresponding to the main face of the casing which allows access to the inside of the channel.

The two facing surfaces parallel to one another are arranged inside the channel. These surfaces delimit the walls of the channel such that the open outer area for the entry and exit of the duct to be regulated is arranged on one side of such facing surfaces and another surface configuring the bottom of the channel delimiting the insertion of the duct is arranged on the opposite side.

According to this configuration, the duct, in its operating position housed inside the channel, is located between the two facing surfaces parallel to one another. The presence of a plurality of protrusions distributed on these facing surfaces gives rise to a channel having a configuration in which the protrusions project towards the duct, pressing on it. This force exerted on the duct by means of the protrusions ensures the correct positioning of the duct, and they also establish a friction force which allows relative retention between the casing and the duct. For example, this retaining strain allows the regulator to be supported by the duct itself without requiring an additional support.

According to another more specific embodiment applicable to the preceding example, *the device is characterized in that the plurality of protrusions are distributed in an alternating manner according to the first direction on the two facing surfaces, such that in a first position according to the first direction of the channel there is a protrusion on the first surface and there is no protrusion on the second surface opposite the first surface, and in a second position adjacent to the first position according to the first direction there is no protrusion on the first surface and there is indeed a protrusion on the second surface, and so on and so forth.*

According to this embodiment, taking into consideration the first direction, the protrusions are arranged in different positions and comprise protrusions on both sides in an alternating manner. Since the protrusions on either side do not coincide, these protrusions do not establish a significant constriction of the duct when it is operatively housed in the channel but do indeed exert force, favoring axial retention in the direction of the duct, also taking advantage of the certain degree of bending strength of the duct.

According to another embodiment applicable to the examples describe above, *the device is characterized in that the regulator element comprises a rotary body rotating about the second direction and the rotary body being provided internally with a through hole in the second direction in which the press element is inserted and arranged in the operating mode, and wherein the movement of the regulator element in the second direction is prevented.*

According to this embodiment, the regulator element comprises a rotary body and establishes a link with the regulator element such that the final configuration is very compact. The rotary body houses therein the press element, reducing the required space. Since the movement of the rotary body in the second direction is prevented, it serves as a support for the press element. That is, when the press element is exerting a constriction force on the elastically deformable duct, the reaction of the duct is transmitted to the press element which in turn transmits this reaction to the rotary body, and since the movement of the rotary body in the second direction is prevented, then the duct is kept constricted without the possibility of varying the degree of constriction.

Throughout the description, it shall be understood that the rotary body has the attribute of rotating mainly with respect to the casing, unless another reference is specifically indicated.

According to another embodiment applicable to the examples describe above, *the device is characterized in that the press element comprises a rod with an outer thread and the rotary body comprises an inner thread adapted to be screwed onto the outer thread of the press element.*

The link between the rotary body and the press element is established through a thread. If the rotary body does not rotate, then any axial force on the press element is transmitted to the rotary body and vice versa. However, in this specific embodiment screwing both elements to one another is what allows modifying the relative position, and therefore the position of the press element which establishes greater or lesser deformation on the elastically deformable duct in order to determine the degree of constriction of said duct.

Furthermore, the press element is configured in the form of a rod movable according to its axial direction through a threaded passage of the rotary body. This axial direction of the rod coincides with the second direction.

According to another more specific embodiment applicable to the preceding example, *the device is characterized in that the press element is provided in at least one outer part with a thread-free flat surface adapted to slide over one or more flat faces provided in the casing to prevent the rotation of the rod and only to allow the axial movement thereof in the second direction.*

According to this embodiment, the user acts on the regulator element only through the rotary body. The relative movement between the rotary body and the rod occurs because the rod is prevented from rotating.

The specific way in which the rod is retained against rotating with respect to the casing is by means of the addition of a flat surface which is in contact with another flat surface of the casing. In this way, the two parts, i.e., the rod and casing, have the capacity of having a relative movement of displacement, but not of rotation dictated by the sliding of the two flat contacting surfaces. Furthermore, given that the rod can only move along the second direction, then the rod does not rotate but is indeed movable in the second direction. Any rotation of the rotary body will therefore cause the movement of the rod in the second direction as a result of the interaction of the outer thread of the rod and the inner thread of the rotary body to which it is coupled. This movement of the rod involves the movement of the press element in the second direction with a forward and backward advancement established by the angular position of the rotary body, and therefore allowing the position of the press element to be regulated.

According to another embodiment applicable to the examples describe above, *the device is characterized in that the rod comprises a tip at one of its ends, the end which presses on the duct, wherein the tip is in the form of a wedge ending in a vertex extending according to a preferably straight line, to secure the support of the duct of the drip along said line.*

According to this embodiment, the press element is supported by means of the end of the rod configuring said press element.

This end is wedged and establishes a support line intended to be the line through which force is exerted on the elastically deformable duct to constrict same.

Given that constriction is carried out along a line, this line is preferably straight so as to be the shortest possible, minimizing the strain required on the duct, and also transverse to the first direction, also for optimizing the region on which force is exerted on the duct. Reduction of contact length also allows the strain required for constricting the duct to be reduced.

According to another embodiment applicable to the examples describe above, *the device is characterized in that the casing comprises on an outer surface at least a first window to allow a part of the regulator element to be accessed from outside the casing, so as to enable the actuation of the regulator element by rotation.*

According to this embodiment, the casing has a window which allows access to the rotary body. This is the way in which the user can exert a rotational force on the rotary body, achieving the exact position of the press element in order to determine a targeted fluid flow rate in the duct. According to another embodiment, this window even allows the rotary element to project out of the casing, making access easier, and also allows the rotary body to be rotated, in each user interaction, at a greater angle and with less effort.

According to another more specific embodiment applicable to the preceding example, *the device is characterized in that the casing further comprises a second window, the first window and the second window being located on opposite faces of the casing to allow a part of the regulator element to be accessed from outside and from both sides of the casing, so as to enable the actuation of the regulator element through both sides of the casing.*

The existence of two windows which allow the user to interact with the rotary body has two advantages, one being the fact that the rotary body is accessible through one window or the other window, so that a nurse or user who is holding several objects in their hands can interact with the device under difficult conditions. The second advantage is that the user can interact with the rotary body from both windows at the same time, for example, by pressing on both diametrically opposed sides with his/her hand such that a higher rotational torque can be exerted with much less effort and with greater comfort. This is the case of using one hand to hold the casing and the other hand to act on the rotary body.

According to another embodiment applicable to the examples describe above, *the device is characterized in that the* casing comprises a passage adapted for the exit of the rod towards the channel.

The passage of the rod towards the channel allows the rod and all the mechanical elements related thereto to be housed in and protected by the casing, allowing only the rod which interacts with the duct, even just its end, to come out. This configuration allows keeping the mechanical elements protected, preventing the entry of fluids or blood which may be found in the environment of use and which may give rise to hygiene problems.

A second aspect of the invention relates to a system for monitoring the fluid flow rate flowing through a duct of a drip, in addition to allowing the capacity to regulate said flow rate.

*The system for monitoring the fluid flow rate comprises:*
- *a device for regulating the fluid flow rate in a drip according to any of the examples described above, and*
- *a measuring device for monitoring the fluid flow rate circulating through the duct;*
*wherein, in the operating mode, the device for regulating the flow in the drip acts on the duct, and the device for monitoring the fluid flow rate performs a reading in the drip comprising the same duct.*

Given that the user has a regulator device like any of those described, as well as a flow rate measuring device which allows monitoring the flow rate passing through the duct, the user can adjust the flow rate while observing the measuring device, thereby adjusting the flow rate to the target flow rate.

The use of a compact measuring device which the user can carry on him/herself and is couplable to the drip is of special interest. Therefore, the way of regulating the regulator only requires temporarily coupling the measuring device on the drip and acting on the regulator device while it is operatively coupled on the duct which is in fluidic communication with the drip which is in turn being monitored. In these conditions, acting on the regulator device allows the flow rate to be adapted to the preestablished target flow rate by simply observing variation in the measurement of the measuring device while acting on the regulator device.

A third aspect of the invention relates to a method for regulating the fluid flow rate flowing through a duct of a drip using the device for regulating the fluid flow rate in a drip according to any of the described examples.

*The method for regulating the fluid flow rate comprises the following steps:*
- *inserting the duct of the drip into the channel of the device for regulating fluid flow rate; and*
- *modifying the regulator element of the device for regulating the fluid flow rate circulating through a duct according to a specific preestablished value, establishing a degree of force of the press element on a part of the duct which constricts to a greater or lesser extent the section of the pressed part of the duct, and accordingly the fluid flow rate in said section of the duct is adjusted according to the preestablished flow rate value.*

More specifically, *the method further comprises coupling a measuring device to the drip for monitoring the fluid flow rate flowing through the duct and modifying the regulator element so that the preestablished flow rate corresponds with the flow rate of the measuring device.*

### DESCRIPTION OF THE DRAWINGS

These and other features and advantages of the invention will be shown more clearly based on the following detailed description of a preferred embodiment, given solely by way of illustrative and non-limiting example in reference to the attached figures:
Figure 1 schematically shows the combination of a drip, a flow rate meter with a sensor which performs measurements on the drip, an outlet duct of the drip shown by means of a dotted line, and a preferred embodiment of a regulator device according to the invention.
Figure 2 shows a perspective view of an embodiment of the regulator device of the preceding figure, the regulator device being formed by a casing in two coupleable parts, in which these two coupleable parts are separated in order to be able to view the inside thereof.
Figure 3 shows the same embodiment with the coupleable parts of the casing, in which both parts are separated but viewed from the opposite side.
Figures 4A and 4B show the combination of the rotary body and the press element seen in a perspective view and from different points of view.
Figure 5 shows the perspective view of only the rotary body.
Figure 6 shows the perspective view of only the press element.

### DETAILED DESCRIPTION OF THE INVENTION

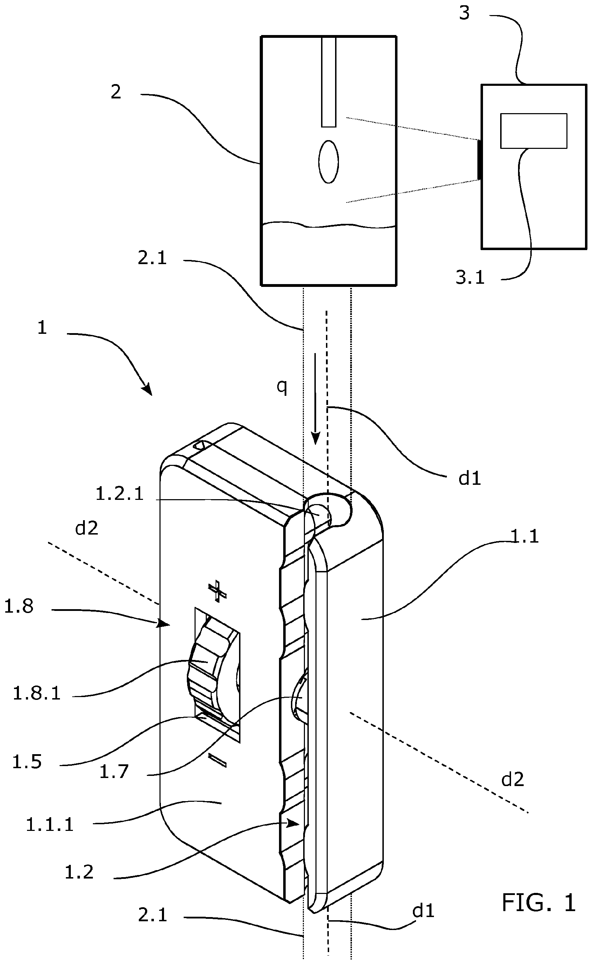

Figure 1 shows a schematic depiction of a drip (2) fed by a reservoir, not shown in this figure, which in turn feeds a duct (2.1) intended for providing a fluid, such as serum or a liquid medicinal product, to a patient. This first schematic figure also shows a measuring device (3) for monitoring the fluid flow rate (q) passing through the duct (2.1) by counting the droplets that fall per unit of time and using an estimate of the volume of each droplet. The measurement result is shown on a display screen (3.1) which allows reading by a user, typically a nurse or a doctor in this case.

The bottom of Figure 1 depicts a preferred embodiment of a device identified as a regulator or a device (1) for regulating flow rate (q), according to the present invention.

The regulator device (1) is formed mainly by a casing (1.1) comprising a channel (1.2) extending according to a first direction (d1). In this figure, taking into consideration the orientation in which the regulator device (1) is shown, the first direction and the channel (1.2) extend according to a vertical direction. The channel (1.2) is an open channel such that it allows the entry of the duct (2.1) originating from the drip (2).

The regulator device (1) according to this embodiment adopts a specific prismatic configuration, with a first front face (1.1.1) and a second rear face (1.1.2), and wherein these faces (1.1.1, 1.1.2) have a rectangular shape with rounded edges. The channel (1.2) in this embodiment opens towards the front face (1.1.1).

It can be seen in this embodiment that the section of the channel is configured for receiving the duct (2.1) having a circular section, but the inlet groove of the open channel (1.2) is narrowed so that the duct (2.1) is forced into same, ensuring that the duct does not come out once inserted unless certain force is applied.

In this way, once the duct (2.1) is inserted into the channel (1.2), it allows the device (1) for regulating flow rate to be arranged in the operating mode and with the capacity to regulate the flow rate of the duct (2.1) by constricting same to a greater or lesser extent.

The casing (1.1) is also formed by two coupleable parts which can be molded separately, such that the movable components are installed therein and both coupleable parts are coupled and attached to configure the regulating device (1).

This first figure also shows a rotary body (1.8) emerging from a first window (1.5) which acts by constricting the duct (2.1) to a greater or lesser extent. The "+" and "-" signs which can be seen in the outer part of the casing (1.1) offer guidance to the user as to the direction of rotation of the rotary body (1.8) depending on whether the user intends to increase or reduce the flow rate (q).

Some details are also seen in this same Figure 1 without the parts having to be separated in order to allow viewing the inside thereof. The way in which the press element (1.7) emerges can be seen through the groove or opening which provides access to the channel (1.2). The press element (1.7) is removable in a second direction (d2) which, according to this preferred example, is perpendicular to the first direction (d1). The press element (1.7) is partially housed inside the casing (1.1) but emerges through a passage (1.4) communicating the inside of the casing (1.1) and the inside of the channel (1.2), providing direct access to the duct (2.1) in order to press on a part of said duct.

Likewise, Figure 1 shows a set of protrusions (1.2.1) intended for pressing on the duct (2.1), favoring its retention.

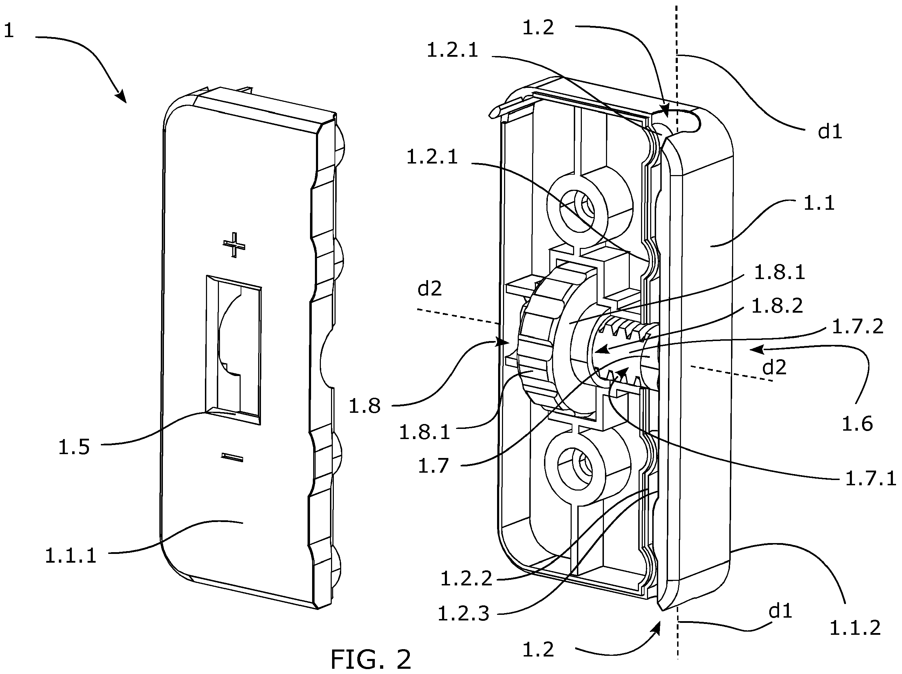

Figure 2 shows a perspective view of the same device (1) for regulating flow rate (q) as Figure 1, in which the two coupleable parts of the casing (1.1) are separated and spaced apart from one another to allow showing the inside thereof.

The coupleable part shown on the left side of Figure 2 shows a first window (1.5) which allows the passage of a rotary body (1.8.1), part of the regulator element (1.8) which allows the exact position of the press element (1.7) to be established.

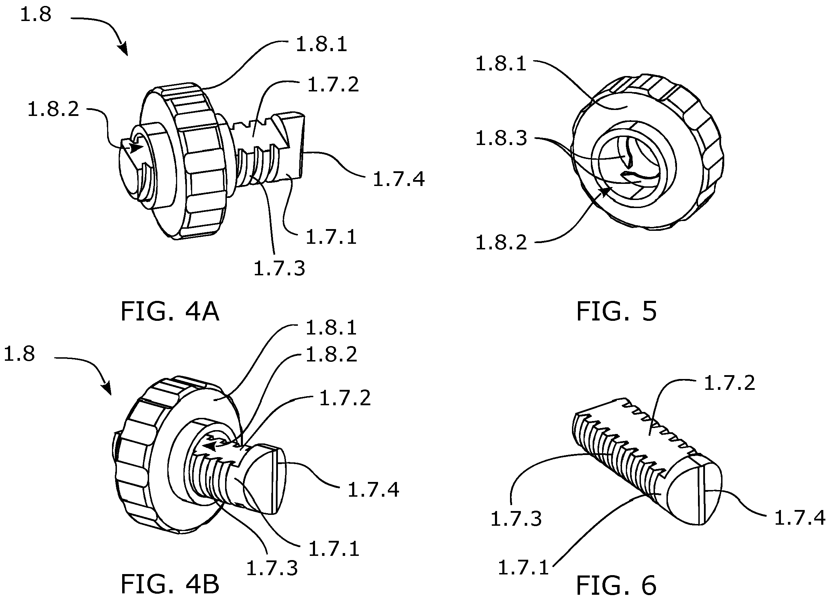

The configuration of this rotary body (1.8.1) consists of a body generated by revolution with the exception that the outer part shows notches which make it easier for the user's fingers not to slip when rotating the rotary body (1.8.1), and also with the exception of an inner thread (1.8.3) which is shown in greater detail in Figure 5. The inner thread (1.8.3) in this embodiment is a single turn thread. The same Figure 5 shows the rotary body (1.8.3) extended according to an axial bushing which gives rise to a through hole (1.8.2) having a length greater than the part shown with a larger diameter. This bushing allows greater stability and axial guiding of the press element (1.7).

Figure 6 shows, in greater detail, this press element (1.7) which is formed by a threaded rod (1.7.1), wherein the outer thread (1.7.3) is not continuous given that it is interrupted by a flat surface (1.7.2) parallel to the geometric axis of the rod (1.7.1) and spaced apart from same.

Figures 4A and 4B are details of the assembly formed by the rotary body (1.8.1) and the press element (1.7), where the rotation of one with respect to the other causes a relative axial movement.

Returning to Figure 2, this assembly is housed in the other coupleable part of the casing (1.1), and where this other coupleable part contains a specific housing configured for housing said assembly.

The configuration of this housing is formed by walls configured from a projection over the flat inner face of the other coupleable part of the silhouette of the assembly formed by the press element (1.7) and the rotary body (1.8.1) by extrusion perpendicular to the flat inner face. The housing of the assembly formed by the press element (1.7) and the rotary body (1.8.1) therefore causes the rotary body (1.8.1) to only be allowed to rotate and the press element (1.7) to only be allowed to perform the movement dictated by the rotary body (1.8.1) when rotated.

The two coupleable parts are attached by clipping, by a screwed attachment, or by both, according to various embodiments.

The same Figure 2 shows the channel (1.2) mainly configured in one of the coupleable parts of the casing (1.1) such that the open channel (1.2) is almost entirely configured in one of the coupleable parts of the casing (1.1) and one of the edges of the groove of the opening corresponds to the edge of the other coupleable part of the casing (1.1) when both coupleable parts are coupled.

This same Figure 2 shows a first surface (1.2.2) and a second surface (1.2.3) arranged facing and separated from one another, both configuring the side walls of the channel (1.2). In this way, the inlet and outlet groove of the duct (2.1) in the channel is arranged on one side of both surfaces (1.2.2, 1.2.3) and the bottom of the channel is arranged on the other side of the surfaces.

Protrusions (1.2.1) intended for exerting force on the duct (2.1) when it is inserted into the channel (1.2) in the operating mode are located on these surfaces. Preferably, the protrusions are distributed in an alternating manner on the first surface (1.2.2) and on the second surface (1.2.3) along the first direction (d1).

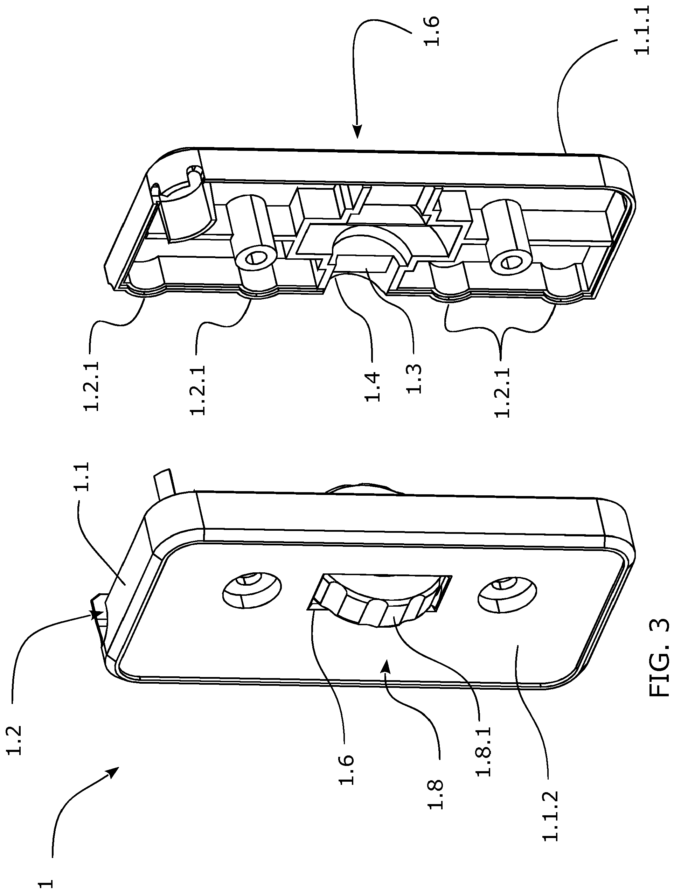

Figure 3 shows a passage (1.4) for the rod (1.7.1) of the press element (1.7) communicating the inside of the casing which houses all the removable elements that allow the axial position to be determined according to the second direction (d2) of the press element (1.7), and the inside of the channel (1.2) in order to be able to exert force on the duct (2.1).

The force of the press element (1.7) is established along a line, shown at end (1.7.4) of the rod in Figure 6, resulting from a wedged termination. This support line is intended for pressing according to a line transverse to the first direction (d1), constricting the passage through the duct (2.1) according to the axial position according to the second direction (d2) of the rod (1.7.1) of the press element (1.7).

The relative rotation between the rotary body (1.8.1) and the rod (1.7.1) is caused by a user who rotates the rotary body (1.8.1) given that the rod does not rotate with respect to the main axis of the outer thread (1.7.3) of the rod because the flat surface (1.7.2) of the rod rests on a complementary surface located in the coupleable part which has been separated.

Since this coupleable part now has its inner face, Figure 3 is a figure which shows the same assembly but from the opposite side. This view shows part of the housing of the assembly formed by the press element (1.7) and the rotary body (1.8.1) but corresponding to the side of the other coupleable part of the casing (1.1), and also the flat face (1.3) which prevents the rotation of the rod so that the rotation of the rotary body (1.8.1) only causes an axial movement according to the second direction (d2).

This second view of Figure 3 also allows showing a second window (1.6) such that the rotary body (1.8.1) emerges and protrudes from the casing (1.1) through the first window on the front face (1.1.1) and through the second window on the rear face (1.1.2).

With this configuration, the rotary body (1.8.1) is accessible from both sides simultaneously, facilitating rotation with respect to the casing (1.1).

Both coupleable parts show a perimeter flange defining a flat recess to receive a plate or an adhesive sheet which allows the screws to be concealed, although these plates, not shown in the figure, must also incorporate the first window (1.5) and the second window (1.6).

## Claims

1. Device (1) for regulating the fluid flow rate (q) of a drip (2), wherein the drip (2) comprises an elastically deformable duct (2.1) through which the fluid flow rate (q) flows, the device (1) comprising:
- a casing (1.1), comprising a channel (1.2) extending along a first direction (d1), configured for housing the duct (2.1) therein in the operating mode,
- a press element (1.7), movable in a second direction (d2), preferably transverse to the first direction (d1), wherein the press element (1.7) is configured for pressing on at least one part of the duct (2.1) when the duct (2.1) is arranged in the operating mode inside the channel (1.2), and
- a regulator element (1.8), connected to the press element (1.7), wherein the regulator element (1.8) is configured for regulating the position of the press element (1.7) in the second direction (d2).

2. Device (1) according to claim 1, **characterized in that** the press element (1.7) is adapted, in the operating mode, to press on at least one part of the duct (2.1), and wherein the channel (1.2) comprises a support surface for the part located opposite the pressed part of the duct (2.1), when the duct (2.1) is arranged in the operating mode in the channel (1.2), to cause a reduction in the section of the duct (2.1) depending on the degree by which the press element (1.7) and the support surface approach one another.

3. Device (1) according to any of the preceding claims, **characterized in that** the press element (1.7) is arranged in an intermediate position of the casing (1.1) with respect to the first direction (d1) in order to press, in the operating mode, on an intermediate part of the duct (2.1) which is arranged in the channel (1.2).

4. Device (1) according to any of the preceding claims, **characterized in that** the channel (1.2) comprises an inner area for the seating of the duct (2.1) when the duct (2.1) is housed in the operating mode in the channel (1.2) and an open outer area forming an opening adapted to receive the duct (2.1) by insertion.

5. Device (1) according to claim 4, **characterized in that** the channel (1.2) comprises in the open outer area two facing surfaces (1.2.2, 1.2.3) parallel to one another, arranged at a distance, and wherein the facing surfaces (1.2.2, 1.2.3) are provided with a plurality of protrusions (1.2.1) distributed according to the first direction for the correct positioning and retention of the duct (2.1).

6. Device (1) according to claim 5, **characterized in that** the plurality of protrusions (1.2.1) are distributed in an alternating manner according to the first direction on the two facing surfaces (1.2.2, 1.2.3), such that in a first position according to the first direction (d1) of the channel (1.2) there is a protrusion (1.2.1) on the first surface (1.2.2) and there is no protrusion on the second surface (1.2.3) opposite the first surface (1.2.2), and in a second position adjacent to the first position according to the first direction there is no protrusion (1.2.1) on the first surface (1.2.2) and there is indeed a protrusion on the second surface (1.2.3), and so on and so forth.

7. Device (1) according to any of the preceding claims, **characterized in that** the regulator element (1.8) comprises a rotary body (1.8.1) rotating about the second direction (d2) and the rotary body (1.8.1) being provided internally with a through hole (1.8.2) in the second direction (d2) in which the press element (1.7) is inserted and arranged in the operating mode, and wherein the movement of the regulator element (1.8) in the second direction (d2) is prevented.

8. Device (1) according to any of the preceding claims, **characterized in that** the press element (1.7) comprises a rod (1.7.1) with an outer thread (1.7.3) and the rotary body (1.8.1) comprises an inner thread (1.8.3) adapted to be screwed onto the outer thread (1.7.3) of the press element (1.7).

9. Device (1) according to the preceding claim, **characterized in that** the press element (1.7) is provided in at least one outer part with a thread-free flat surface (1.7.2) adapted to slide over one or more flat faces (1.3) provided in the casing (1.1) to prevent the rotation of the rod (1.7.1) and only to allow the axial movement thereof in the second direction (d2).

10. Device (1) according to claim 8 or 9, **characterized in that** the rod (1.7.1) comprises a tip (1.7.4) at one of its ends, the end which presses on the duct (2.1), wherein the tip (1.7.4) is in the form of a wedge ending in a vertex extending according to a preferably straight line, to secure the support of the duct (2.1) of the drip (2) along said line.

11. Device (1) according to any of the preceding claims, **characterized in that** the casing (1.1) comprises on an outer surface at least a first window (1.5) to allow a part of the regulator element (1.8) to be accessed from outside the casing (1.1), so as to enable the actuation of the regulator element (1.8) by rotation.

12. Device (1) according to the preceding claim, **characterized in that** the casing (1.1) further comprises a second window (1.6), the first window (1.5) and the second window (1.6) being located on opposite faces of the casing (1.1) to allow a part of the regulator element (1.8) to be accessed from outside and from both sides of the casing (1.1), so as to enable the actuation of the regulator element (1.8) through both sides of the casing (1.1).

13. Device (1) according to any of the preceding claims, **characterized in that** the casing (1.1) comprises a passage (1.4) adapted for the exit of the rod (1.7.1) towards the channel (1.2).

14. System for monitoring the fluid flow rate flowing through a duct (2.1) of a drip (2), the system comprising:
- the device (1) for regulating the fluid flow rate in a drip (2) according to any of claims 1 to 13, and
- a measuring device (3) for monitoring the fluid flow rate (q) circulating through the duct (2.1);
wherein, in the operating mode, the device (1) for regulating the flow in the drip (2) acts on the duct (2.1), and the device (3) for monitoring the fluid flow rate (q) performs a reading in the drip (2) comprising the same duct (2.1).

15. Method for regulating the fluid flow rate flowing through a duct (2.1) of a drip (2) using the device (1) for regulating the fluid flow rate (q) in a drip (2) according to any of claims 1 to 13, wherein
- the duct (2.1) of the drip (2) is inserted into the channel (1.2) of the device (1) for regulating the fluid flow rate (q); and
- the regulator element (1.8) of the device (1) for regulating the fluid flow rate (q) circulating through a duct (2.1) is modified according to a specific preestablished value, establishing a degree of force of the press element (1.7) on a part of the duct (2.1) which constricts to a greater or lesser extent the section of the pressed part of the duct (2.1), and accordingly the fluid flow rate in said section of the duct (2.1) is adjusted according to the preestablished flow rate value.

16. Method according to the preceding claim, which further comprises coupling a measuring device (3) to the drip (2) for monitoring the fluid flow rate (q) flowing through the duct (2.1) and modifying the regulator element (1.8) so that the preestablished flow rate corresponds with the flow rate of the measuring device (3).
